# EUROPEAN PATENT APPLICATION

(11) **EP 1 449 898 A1**
(43) Date of publication of application: **25.08.2004**
(21) Application number: 03100428.6
(22) Date of filing: 21.02.2003
(51) Int. Cl.: C09J 153/02, C09J 7/02, C09J 7/04, C09J 153/00, A61F 13/58, A61L 15/58

(54) **Adhesive composition and tapes and labels derived therefrom**

(71) Applicant: KRATON Polymers Research B.V., 1031 CM Amsterdam (NL)
(72) Inventor: de Keyzer, Noel, B-1348, Ottignies - Louvain-La-Neuve (BE)
(74) Representative: Beetz, Tom

(57) **Abstract**

Adhesive composition for tapes, labels and bandages to be used at temperatures of +5°C and lower, comprising
a) at least one block copolymer, comprising at least two terminal blocks of poly(vinyl aromatic compound) and at least one midblock of a randomly copolymerized mixture of isoprene and butadiene, optionally mixed with a diblock copolymer comprising one poly(vinyl aromatic compound) block and one randomly copolymerized mixture of isoprene and butadiene, and optionally mixed with a block copolymer, comprising at least one block of poly(vinyl aromatic compound) and at least one block of poly(butadiene) or poly(isoprene),
b) at least one mixed aliphatic/aromatic hydrocarbon resin or a blend of aliphatic and aromatic hydrocarbon resins, having an aromatic H-NMR content between 6 and 22%, and
c) a plasticizer in an amount of at most 25 wt.%, relative to the weight of the adhesive composition,
and tapes, labels and bandages comprising said adhesive compositions, applied on a substrate layer, and the use of said tapes, labels or bandages.

## Description

### Field of the invention

An adhesive composition comprising
i. one or more styrenic block copolymers
ii. one or more tackifier resins, and
iii. one or more plasticizers.

### Background of the invention

Adhesive compositions based on styrenic block copolymers as thermoplastic elastomer components are well known in the art.

These compositions are for instance used in pressure sensitive adhesive (PSA) for industrial tapes, packaging tapes, labels and bandages (e.g. plasters).

More in particular styrene-isoprene-styrene block copolymers (S-I-S) and styrene-butadiene-styrene block copolymers (S-B-S) are widely used in these adhesive compositions.

Both classes of block copolymers give the adhesive compositions specific properties related to the respective inherent characteristics of these block copolymers.

For example, the softness of S-I-S makes this polymer type the material of choice for pressure sensitive applications in tapes and labels, while the elevated cohesion of S-B-S makes this material attractive for construction adhesives for disposable soft goods.

S-I-S block copolymers have until now successfully been applied in industrial and packaging tape and label applications.

Although such S-I-S block copolymer containing compositions showed outstanding tack, peel and cohesion at room temperature, i.e. at temperatures around 20°C, they have appeared to lack adequate adhesive properties in cold environments, namely 5°C and below.

It is well known from e.g. Handbook of Pressure Sensitive Adhesive Technology, Don Satas, Chapter 13, Thermoplastic Rubbers, A-B-A block copolymers, p 367 (1989, 2^{nd} ed.), that most adhesive compositions based on S-I-S block copolymers loose their tack and adhesion properties as temperature decreases from about 15°C.

It was possible to formulate adhesive compositions based on S-I-S block copolymers, having a good tack below 15°C, but in order to achieve an acceptable compromise with the adhesive properties, relatively low proportions of high softening point hydrocarbon resins and relatively high proportions of plasticizers are needed.

However, high proportions of plasticizer have the disadvantages (a) that the peel and cohesion of the adhesive is reduced and (b) that the plasticizer migrates out of the formulation, modifying the visual and property appearance of the final products, like oily spots in a paper label.

During the splitting of roll products, too much oil generates edge oozing, provoking that two adjacent rolls stick together.

Therefore there is a strong need for adhesive compositions which keep good tack, adhesion and cohesion at low temperatures, and more in particular at temperatures in the range from +5 to -25°C, which enables the manufacture of tapes and labels to be used in cold environments e.g. some electrical insulating tapes or on frozen articles, e.g. food and medicines in deep freezers.

It is an object of the present invention to provide adhesive compositions which keep good tack, adhesion and cohesion at low temperatures, and which do not contain high amounts of plasticizers.

Another object of the present invention is formed by labels and tapes, which retain adequate properties when stored for a long time at low temperatures.

Another object of the present invention is to provide adhesive compositions, which enable the manufacture of repositionable or removable tapes, labels and bandages (e.g. plasters), which can be used at low temperatures.

As result of extensive research and experimentation, said adhesive compositions aimed at, and labels, tapes and bandages to be manufactured from them, have now surprisingly been found.

### Summary of the invention

Accordingly the present invention relates to an adhesive composition for tapes, labels and bandages to be used at temperatures of +5°C and lower, comprising
a) at least one block copolymer, comprising at least two terminal blocks of poly(vinyl aromatic compound) and at least one midblock of a randomly copolymerized mixture of isoprene and butadiene, said block copolymer being optionally mixed with a diblock copolymer, comprising one poly(vinylaromatic) block and one randomly copolymerized mixture of isoprene and butadiene, and optionally mixed with a block copolymer, comprising at least one block of poly(vinyl aromatic compound) and at least one block of poly(butadiene) or poly(isoprene),
b) at least one mixed aliphatic/aromatic hydrocarbon resin or a blend of aliphatic and aromatic resins, having an aromatic H-NMR content between 6 and 22 %, and
c) a plasticizer in an amount of at most 25 wt.%, relative to the weight of the adhesive composition,
and to tapes, labels and bandages derived from it.

### Detailed description of the invention

The main block copolymer component used in the adhesive composition is a block copolymer, having a structure represented by the general formulae S-(I/B)-S (1) or [S-(I/B)]ₙX (2), optionally mixed with a diblock copolymer S-(I/B), and optionally mixed with minor amounts of one or more block copolymers, selected from the group S-B, S-B-S, S-I and S-I-S, wherein S represents a poly(vinyl aromatic compound) block, (I/B) represents a block of a randomly copolymerized mixture of isoprene and butadiene, wherein the weight ratio between isoprene and butadiene is in the range of from 70:30 to 30:70, or in a mole/mole ratio of from 1.1/0.55 to 0.45/1.3, wherein B represents a poly(butadiene) block, wherein I represents a poly(isoprene) block, wherein n is an integer equal to or greater than 2, and wherein X is the residue of a coupling agent.

Preferred weight ratios between isoprene and butadiene are in the range of from 60:40 to 30:70 or in a molar ratio of from 0.89/0.75 to 0.45/1.3.

As an example of the aromatic vinyl compound useful in the practice of the present invention, may be mentioned styrene, alpha-methylstyrene, p-methylstyrene, o-methylstyrene, p-tert.butylstyrene, dimethylstyrene, and vinyl naphthalene or mixtures thereof. Of these, styrene is particularly preferred from the viewpoints of easy availability, reactivity, physical properties of the resulting block copolymers. The A polymer block may contain minor amounts of comonomers other than an aromatic vinyl compound, e.g., up to 5 wt.% of a copolymerizable monomer such as butadiene and/or isoprene (based on the weight of the total block). Most preferred are A blocks derived from substantially pure styrene.

These polymer blocks A preferably have a true molecular weight in the range from 9,500 to 25,000.

The mixed polymer midblock (I/B) is made of butadiene and isoprene as copolymerizing monomers, although it too may contain minor amounts of other comonomers, e.g. up to 5 wt.% of a copolymerizable monomer such as styrene (based on the weight of the total block), but mixtures of substantially pure isoprene and butadiene are preferred.

In the block copolymers according to the present invention, the proportion of bound aromatic vinyl compound is in the range of 10-50 wt.%, preferably 15-45 wt.% based on the total block copolymer. The proportion of bound butadiene is 18-80 wt.%, preferably 40-70 wt.% in total. The proportion of bound isoprene is 15-70 wt.%, preferably 30-70 wt.%. These amounts of bound monomers (plus copolymerizable monomers, if any) add up to 100 wt.%.

The block copolymers to be applied in the adhesive compositions according to the present invention each preferably have a weight average molecular weight (Mw, expressed in terms of polystyrene) ranging from 100,000 to 500,000, preferably from 150,000 to 250,000 as determined by gel permeation chromatography (GPC, using the method described by J. R. Runyon et al, J. Polym. Sci., 13, 2359 (1969).

The block copolymers to be applied in the adhesive compositions according to the present invention each preferably contain 1,2-vinyl bonds and/or 3,4-vinyl bonds in a proportion in the range of from 5 to 40 wt.% based on the weight of the conjugated diene, and preferably from 5 to 20 wt.%, based on the weight of conjugated diene. The block copolymers according to the present invention preferably each have a storage modulus (G') of 1 to 300 MPa in a visco-elasticity measurement in a temperature range of from 0 to 50°C, and only one peak on loss tangent (tan δ) attributable to the mixed butadiene/isoprene polymer block at a temperature of -50°C or below. When a block copolymer having a storage modulus (G') lower than 1 MPa is used as a base polymer for a pressure sensitive adhesive, then the holding power of the PSA is lowered. On the other hand, any storage modulus exceeding 300 MPa results in a pressure sensitive adhesive lowered in tackiness.

Said block copolymers to be applied as main component (a) in the adhesive composition, have a randomly copolymerized block (I/B), which means that the mixed midblock shows no significant single homopolymer block formation.

They can be prepared as described in WO 02/057386.

More in particular, polymers having mixed midblocks may be defined as having average homopolymer block lengths of less than 100 monomer units, preferably less than 50 monomer units, more preferably less than 20 monomer units.

Average homopolymer block length may be determined by carbon-13 NMR, as described in detail in WO 02/057386, which is herein incorporated by reference.

The block copolymers according to the present invention can be made e.g. by coupling living diblock copolymer prepared by anionic polymerization with a coupling agent.

As examples of the coupling agent, may be mentioned tin coupling agents such as tin dichloride, monomethyltin dichloride, dimethyltin dichloride, monoethyltin dichloride, diethyltin dichloride, methyltin trichloride, monobutyltin dichloride, dibutyltin dibromide, monohexyltin dichloride and tin tetrachloride; halogenated silicon coupling agents such as dichlorosilane, monomethyldichlorosilane, dimethyldichlorosilane, monoethyldichlorosilane, diethyldichlorosilane, monobutyldichlorosilane, dibutyldichlorosilane, monohexyldichlorosilane, dihexyldichlorosilane, dibromosilane, monomethyldibromosilane, dimethyldibromosilane, silicon tetrachloride and silicon tetrabromide; alkoxysilanes such as tetramethoxysilane; divinyl aromatic compounds such as divinylbenzene and divinylnaphthalene; halogenated alkanes such as dichloroethane, dibromoethane, methylene chloride, dibromomethane, dichloropropane, dibromopropane, chloroform, trichloroethane, trichloropropane and tribromopropane; halogenated aromatic compounds such as dibromobenzene; epoxy compounds such as the diglycidyl ether of bisphenol-A and the like (e.g., "EPON" 825 diglycidyl ether, trademark) and other coupling agents such as benzoic esters, CO, 2-chloropropene and 1-chloro-1,3-butadiene. Of these, "EPON" 825 diglycidyl ether, dibromobenzene, tetramethoxysilane or other tetra(alkoxy)silanes are preferred.

The main block copolymer in component (a) may hence comprise a mixture of the coupled polymer according to the general formulae (1) or (2) and of the intermediate diblock, e.g. in a weight ratio of 100/0 to 30/70.

It will be appreciated that the main block copolymer component (a) may also be formed by a block copolymer obtained by sequential polymerization of batches of the respective monomers (e.g. styrene and mixtures of butadiene/isoprene, optionally in combination with reinitiation, if additional diblock copolymer is desired. The block copolymers of formulae (1) and (2) can be made by mere adaptation of common processes used for the preparation of S-B-S type block copolymers and/or S-I-S type block copolymers, using a mixture of butadiene/isoprene instead. Of importance in the preparation of the block copolymers according to the present invention is to avoid homopolymer block formation, to ensure appropriate B/I ratio, and to produce a polymer block wherein the random midblock has a Tg of -50°C or less. Generally no randomizer will be used.

As specified hereinbefore, the main block copolymer of formulae (1) and (2), which usually will comprise corresponding diblocks, can be mixed with minor proportions of conventional diblock copolymers and/or triblock copolymers, comprising poly(vinyl aromatic compound) blocks and poly(butadiene) blocks or poly(isoprene) blocks in a proportion of from 0 to 50 wt.%, relative to the weight of component (a) and preferably in a proportion of from 0 to 30 wt.%.

More preferably said diblock copolymers and triblock copolymers have been obtained in one process, comprising the preparation of an initial living diblock, which can be subsequently coupled to a triblock copolymer by means of a coupling agent as specified hereinbefore.

It will be appreciated that the diblock copolymer and/or triblock copolymers, which can optionally be incorporated in component (a), may have apparent molecular weights which are about the half of those of the main block copolymer component for an additional copolymer diblock and about the same as those of the main block copolymer for an additional triblock copolymer respectively.

### Component (b)

Suitable tackifying resins or mixtures of resins have been found to have an aromatic H-NMR content between 6 and 22 %, and preferably from 9 to 22 %, and more preferably from 9 to 18 %.

They can be selected from modified aliphatic hydrocarbon resins such as modified C5 hydrocarbon resins (C5/C9 resins), styrenated terpene resins, partially hydrogenated C9 hydrocarbon resins and mixtures thereof.

More preferred examples of resins to be used as component (b) are: MBG 223, a modified aliphatic hydrocarbon resin, showing a H-NMR aromatic content of 11.3%, a softening point of 88°C, manufactured by HERCULES BV; ESCOREZ 2101, a modified aliphatic hydrocarbon resin, showing a H-NMR aromatic content of 13.15%, a softening point of 93°C, manufactured by EXXON CHEMICALS, and WING TACK 86, a modified hydrocarbon resin, showing a H-NMR aromatic content of 9.6% and a softening point of 86°C, manufactured by GOODYEAR CHEMICALS, QUINTONE P-195, a modified aliphatic hydrocarbon resin having a H-NMR aromatic content of 13%, a softening point of 94°C, manufactured by NIPPON ZEON.

Preferred solid tackifying resins will have softening points in the range of from 85 to 95°C.

The adhesive composition according to the present invention preferably comprises from 50 to 300 parts by weight and more preferably from 100 to 200 parts by weight of tackifying resin per 100 parts by weight of component (a).

In preferred adhesive compositions, the component (b) occurs in a proportion of from 35 to 55 wt.%, relative to the weight of the composition.

### Component (c)

Suitable plasticizers include predominantly plasticizing oils that are paraffinic or naphthenic in character (carbon aromatic distribution ≤ 5%, preferably ≤ 2%, more preferably 0% as determined according to DIN 51378) and a glass transition temperature lower than -55°C as measured by Differential Scanning Calorimetry. Those products are commercially available from the Royal Dutch/Shell Group of companies, like SHELLFLEX, CATENEX, and ONDINA oils. Other oils include KAYDOL oil from Witco, or TUFFLO oils from Arco or NYPLAST from NYNAS. Other plasticizers include compatible liquid tackifying resins like REGALREZ R-1018 or WINGTACK 10. (SHELLFLEX, CATENEX, ONDINA, KAYDOL, TUFFLO, REGALREZ, WINGTACK, NYPLAST are trademarks).

Other plasticizers may also be added, like olefin oligomers; low molecular weight polymers (≤ 30,000 g/mol) like liquid polybutene, liquid polyisoprene copolymers, liquid styrene/isoprene copolymers or liquid hydrogenated styrene/conjugated diene copolymers; vegetable oils and their derivatives; or paraffin and microcrystalline waxes. The composition according to the present invention preferably comprises a plasticizer in a weight proportion of from 5 to 15 wt.%, relative to the weight of the complete composition and of from 10 to 85 parts by weight of plasticizer per 100 parts by weight of block copolymer constituent (a). Also the or each block copolymer of component (a) may be pre-blended with a small amount of plasticizer by the manufacturer of said copolymer.

### Other components (non-limitative)

Other rubber components may be incorporated into the adhesive compositions according to the present invention.

It is also known in the art that various other components can be added to modify the tack, the odor, the color of the adhesives. Antioxidants and other stabilizing ingredients could also be added to protect the adhesive from degradation induced by heat, light and processing or during storage.

Several types of antioxidants can be used, either primary antioxidants like hindered phenols or secondary antioxidants like phosphite derivatives or blends thereof.

Examples of commercially available antioxidants are IRGANOX 565 from Ciba-Geigy (2,4-bis-(n-octylthio)-6-(4-hydroxy-3,5-di-tertiary-butylanilino)-1,3,5-triazine), IRGANOX 1010 from Ciba-Geigy (tetrakis-ethylene-(3,5-di-tertiary-butyl-4-hydroxy-hydrocinnamate)methane) and POLYGARD HR from Uniroyal (tris-(2,4-di-tertiary-butyl-phenyl)phosphite).

Other antioxidants developed to protect the gelling of the polybutadiene segments can also be use, like the SUMILIZER GS from Sumitomo (2[1-(2-hydroxy-3,5-di-ter-pentylphenyl)-ethyl)]-4,6-di-tert-pentylphenylacrylate); SUMILIZER T-PD from Sumitomo (pentaerythrythyltetrakis(3-dodecylthiopropionate)); or mixtures thereof. (IRGANOX, POLYGARD and SUMILIZER are trademarks).

### Preparation of the composition

No particular limitation is imposed on the preparation process of the adhesive composition. Therefore, there may be used any process such as a mechanically mixing process making use of rolls, a Banbury mixer or a Dalton kneader, a hot-melt process characterized in that heating and mixing are conducted by using a melting kettle equipped with a stirrer, like a high shear Z-blade mixer or a single- or twin-screw extruder, or a solvent process in which the compounding components are poured in a suitable solvent and stirred, thereby obtaining an intimate solution of the pressure sensitive adhesive composition.

### Use of the composition

PSA compositions according to the present invention may be applied without using any solvent (e.g., hot-melt) or in the form of their solutions to a base material such as paper or a plastic film by means of a proper coater, thereby producing various kinds of pressure sensitive adhesive tapes for tapes or labels which can be used in cold environments and which can be used for long storage at low temperatures.

During label manufacture, a laminate of a face stock, pressure sensitive adhesive layer and a release liner are passed through an apparatus, which converts the laminate into commercially useful labels and label stock. The process involves, amongst others, die-cutting and matrix stripping to leave labels on a release liner.

It has surprisingly been found that during the manufacture of tapes, labels and bandages according to the present invention, the fouling of knives used in slitting and cutting of roll and sheet stocks is significantly reduced.

It will be appreciated that another aspect of the present invention is formed by the use of tapes, labels or bandages on packed frozen articles such as food, medicines and the like. A more particular aspect is formed by the use of repositionable or removable tapes or labels on frozen articles.

The present invention will hereinafter be illustrated more specifically by the following examples, however without restricting the scope to these specific embodiments.

### Test methods

Standard peel, tack, cohesion and viscosity tests were carried out on these formulations as described in the Test method manual for Pressure Sensitive Tapes from the Pressure Sensitive Tape Council (PSTC), the standard FINAT test method for Pressure sensitive materials, the AFERA test methods for Pressure Sensitive Adhesive Tapes and the ASTM related methods. Different testing surfaces have been used in function of the application: chromed stainless steel plates (No. 304)("ss") as recommended by the FINAT and Kraft paper.
- Rolling Ball Tack (RBT) is the distance, expressed in centimeters, a steel ball rolls on the adhesive film with a standard initial velocity (Pressure Sensitive Tape Council Test No. 6; ASTM D3121-73). Small numbers indicate aggressive tack.
- Loop Tack (LT) was determined using PSTC-5 and FTM 9 loop tack method. High numbers LT indicate aggressive tack. Results are expressed in Newton/25 mm (N/25 mm).
- Peel Adhesion (PA) was determined by Pressure Sensitive Tape Council Method No. 1 and ASTM D3330-83. Large numbers indicate high strength when peeling a test tape from a steel substrate. Results are expressed in N/25 mm.
- Holding Power (HP) is the time required to pull a standard area (2.5 x 1.3 cm) of tape from a standard test surface (steel = ss) under a standard load (1 kg, 2 or 5 kg), in shear at 2° (Pressure Sensitive Tape Council Method No. 7; ASTMD-3654-82). Long times indicate high adhesive strength. Results are expressed in hours (h) or minutes (min). The type of failure mode is expressed as adhesive failure (AF) or cohesive failure (CF). This test can be carried out at room temperature (about 23°C) or at a more elevated temperature, depending on the test.
- The SHAFT (shear adhesion failure temperature) was measured by 2.5 x 2.5 cm Mylar to chromed ss plates with a 1 kg weight. The samples are placed in an oven and the temperature raised by 22°C/minute. SAFT measures the temperature at which the lap shear assembly fails.
- Glass transition temperatures Tg have been determined by Differential Scanning calorimetry with a temperature sweep of 40°C/min. The Tg is measured at the onset of the transition.
- Polystyrene content was determined by ¹H-NMR.
- Average homopolymer block lengths have been determined by ¹³C NMR using the method described herein before. ¹³C NMR spectra of polymer samples were obtained with a Bruker AMX-500 FT spectrometer operating at 125 MHz. Quantitative proton-decoupled spectra were recorded with a 90° ¹³C excitation pulse and a repetition rate of 10 s. 10% (w/w) of polymer solutions in CDCl₃ were used. To improve the relaxation time 0.1 mol/l chromium acetylacetonate was added. The applied line broadening was 2 Hz. The spectra were referenced such that the aliphatic carbons of transpolybutadiene are at 31.9 ppm.
- Low temperature conditions: the tack tests have been carried out in a climate chamber, wherein the temperature could be adjusted down to 0°C. The RBT has been measured at respectively 23, 15, 10, 5 and 0°C. Prior to testing the samples were conditioned at the testing temperature during 24 hours.

### Synthesis of the block copolymers A and B

Cyclohexane, styrene, butadiene and isoprene were purified by activated aluminumoxide and stored at 4°C under a nitrogen atmosphere. EPON 826 diglycidyl ether and dibromoethane (EDB) were used as coupling agent. Prior to synthesis, a monomer mixture of butadiene and isoprene (at a weight/weight ratio given in Table 1) was prepared and stored under nitrogen at 4°C. This mixture was used as such.

An autoclave, equipped with a helical stirrer was charged with cyclohexane and the content was heated to 50 to 60°C. As initiator sec-BuLi was dosed immediately followed by styrene monomer, which was allowed to polymerize to completion. The reaction temperature was increased to 70°C, at which temperature a butadiene/isoprene monomer mixture (B/I) was dosed and reacted. The resulting diblock was coupled with an excess EPON 826 diglycidyl ether or alternatively with an excess of EDB. This excess was optionally scavenged with sec-BuLi and followed by addition of ethanol as terminator. The reaction mixture was cooled to 40°C, transported to a blending vessel and a stabilization package was added (comprising IRGANOX 565 and tris(nonylphenol)phosphite 0.08/0.35 phr as a cyclohexane solution) and stirred at RT. Dry rubber was obtained by steam coagulation finishing, followed by drying in an oven. The polymers were analyzed by GPC according to the method described by J.R. Runyon et al. J. Polym. Sci. 13, 2359 (1969).

Table 1 lists the amounts in which the components have been used. The results of the GPC analysis are in Table 2.

Further components used in the examples are listed in Table 3.

### Synthesis of polymer with sequential/reinitiation

### (Polymer C)

Cyclohexane, styrene, butadiene and isoprene have been purified by activated aluminumoxide and were stored at 4°C under a nitrogen atmosphere.

Prior to synthesis, a monomer mixture of butadiene and isoprene (at the desired weight/weight ratio) was prepared and stored under nitrogen at 4°C. This mixture was used as such.

An autoclave, equipped with a helical stirrer was charged with cyclohexane and the content was heated to 50°C. As initiator sec-BuLi was dosed immediately followed by styrene monomer that was allowed to polymerize to completion. The reaction temperature was increased to 60°C and followed by dosing and reaction to completion of a butadiene/isoprene monomer mixture (B/I). A second portion of sec-BuLi was dosed immediately followed by dosing and reacting to completion of a butadiene/isoprene monomer mixture (B/I). A second portion of styrene monomer was dosed and reacted to completion. The reaction mixture was terminated with an stoichiometric amount of alcohol, cooled to 40°C, transported to a blending vessel and a stabilization package was added and stirred at room temperature.

White polymer was obtained by steam coagulation finishing, followed by drying in a oven.

The polymer was analyzed by GPC according to the method described by J.R. Runyon and al. in J. Polym. Sci., 13, 2359 (1969).

The results have been listed in Table 2.

**Table 1**

| Polymer | A | B | C |
|---|---|---|---|
| Cyclohexane (1) | 77 | 14 | 30 |
| Initiator (mmol) | 20.5 | 27.4 | 28 |
| Styrene (gram) | 300 | 329 | 290 |
| B/I (ratio) | 1.5 | 1 | 1 |
| B/I (gram) | 1490 | 1637 | 1230 |
| Initiator (mmol) | | | 23.5 |
| B/I (gram) | | | 2285 |
| Epon 826(gram) | 0.24 | | |
| EDB (ml) | | 0.56 | |
| Ethanol (ml) | 1 | 1 | 1 |

**Table 2**

| Polymer | A | B | C |
|---|---|---|---|
| Mw Polystyrene *10³ | 10.8 | 10.9 | 10.9 |
| Total Mw *10³ | 188 | 245 | 191 |
| Coupling efficiency % | 71 | 43 | 68 |
| Polystyrene content wt.% | 18 | 17 | 19 |
| B/I ratio | 40/60 | 50/50 | 50/50 |
| Vinyl in B wt.% | 8 | 8 | 8 |
| Vinyl in I wt.% | 5 | 5 | 5 |

Further components used in the tested adhesive compositions have been listed in Table 3.

**Table 3**

| | |
|---|---|
| Kraton D-1160 | is a linear styrene-isoprene-styrene block copolymer with 19% polystyrene content, a total molecular weight of 178 000 g/mole and a coupling efficiency of 100% |
| Kraton D-1113 | is a linear styrene-isoprene-styrene block copolymer with 16% of polystyrene content, a coupling efficiency of 44% and a total molecular weight of 240 000 g/mole |
| Polymer A | is a linear styrene-isoprene/butadiene-styrene block copolymer with 18% polystyrene content, a coupling efficiency of 71%, a total molecular weight of 188 000 g/mole and a isoprene/butadiene w% ratio of 60/40 |
| Polymer B | is a linear styrene-isoprene/butadiene-styrene block copolymer with 17% of polystyrene content, a coupling efficiency of 43%, a total molecular weight of 245.000 g/mole and an isoprene/butadiene wt.% ratio of 50/50 |
| Polymer C | is a linear styrene-(isoprene/butadiene)-styrene block copolymer with a 19 wt.% polystyrene content, a total molecular weight of 191 000 g/mole and a isoprene/butadiene wt.% ratio of 50/50, mixed with a styrene-(isoprene/butadiene) diblock copolymer with a molecular weight of 95 000 g/mole, and a proportion of 30 mol/mole%, relative to the triblock copolymer |
| Kraton D-1118 | is a linear styrene-butadiene-styrene block copolymer with 31% of polystyrene |
| | content, a coupling efficiency of 22% and a total molecular weight of 170 000 g/mole |
| Finaprene 1205 | is a styrene-butadiene diblock copolymer with a polystyrene content of 25%, having a total molecular weight of 120 500 g/mole |
| Piccotac 1094 | is an experimental aliphatic hydrocarbon resin with a softening point of 95°C, a NMR-H aromaticity of 0%, developed by EASTMAN BV |
| MGB 223 | is an experimental aliphatic/aromatic hydrocarbon resin with a softening point of 88°C and a NMR-H aromaticity of 11.3%, developed by EASTMAN BV |
| Wingtack 86 | is an aliphatic/aromatic hydrocarbon resin with a softening point of 86°C and a NMR-H aromaticity of 9.6%, developed by GOODYEAR CHEMICALS |
| Edelex N 956 Edelex SM 925 Irganox | is a naphtenic oil from DEUTSCHE SHELL AG is a paraffinic oil from DEUTSCHE SHELL AG is an anti-oxidant from CIBA |

All the formulations in the examples have been prepared out of solvent. The different ingredients were poured in toluene and mixed for 24 hours to obtain the dissolution. Afterward, the solution have been coated on a Polyester Film (Mylar -36 microns thick) with an automatic Bar Coater to obtain an adhesive coating weight of 22 g/m² dry. Thereafter, the samples have been laminated with a siliconized paper to protect them. Prior to testing, the samples are stored in a conditioned room at 21 °C and 50% relative humidity.

### Example 1:

The adhesive properties of formulations based on a SIS and the polymer A and C (described in Table 1) are compared in Table 4. Particularly, the Rolling Ball Tack has been measured at different temperatures, as low as +5°C in this case.

It is clearly demonstrated that the combination of polymer A and the WINGTACK 86 allows to have good tack properties at low temperature, much better than SIS in the same formulation.

**Table 4**

| **Ingredients** | **units** | **F-1 Comp** | **F-2** | **F-3** |
|---|---|---|---|---|
| Kraton D-1160 | | 100 | | |
| Polymer A | | | 100 | |
| Polymer C | | | | 100 |
| Wingtack 86 | | 110 | 110 | 110 |
| Edelex 956 N | phr | 10 | 10 | 10 |
| Irganox 1010 | | 3 | 3 | 3 |
| | | | | |
| RTB at +23°C | cm | 12 | 1.6 | 4 |
| RTB at +15°C | cm | >40 | 3.4 | 9.5 |
| RTB at +10°C | cm | - | 4 | 25 |
| RTB at + 5°C | cm | - | 23 | |
| | | | | |
| Loop Tack 23°C | N/25mm | 15 | 15 | 16 |
| Peel Adhesion 23°C | N/25mm | 18 | 12 | 13 |
| Holding Power 2 kg-23°C | hours | >100 | 50 | >100 |

### Example 2

The adhesive properties of formulations based on SIS and polymer B (described in Table 1) are presented in Table 5.

The glass transition temperatures were calculated with the help of the Fox Equation (Handbook of Pressure Sensitive Adhesive Technology - Don Satas - 1989- page 369)

The Rolling Ball Tack was measured at different decreasing temperatures down to 0°C.

At the same ingredient ratio and same formulation composition (Formulations F-4 and Formulation F-6), the polymer B allows achieving better tack at lower temperature than the SIS formulation.

If the composition of the SIS formulation (F-4) is adjusted to have the same formulation glass transition temperature Tg of -25°C as that of the F-6 containing the polymer B, then the Formulation F-5 is obtained that contains a higher level of oil, namely 85 phr versus of 40.

This higher amount of oil in Formulation F-5 improved the Rolling Ball Tack values but has the following detrimental effects:
- the other adhesive properties tack, peel adhesion, cohesion and SAFT (Shear Adhesive Failure temperature) are much lower and therefore the adhesive properties are not well balanced.
- the higher oil content will enhance the oil bleeding in the front material, like paper in labels, creating undesirable side effects (oily spots that reduces the aesthetics of the paper surface) and modification of the adhesive properties.
- the higher oil content is also the responsible for the edge oozing of the adhesive because of the lower cohesion, and provokes the mutual sticking of adjacent tape rolls and that of stacked sheets.

Therefore, the polymers of the invention are well designed to develop adhesives with lower service temperature than SIS but with the marked advantage to use less oil or plasticizer.

**Table 5**

| | **units** | **Comp F-4** | **Comp F-5** | **F-6** |
|---|---|---|---|---|
| D-1113 | | 100 | 100 | |
| Polymer B | | | | 100 |
| Wingtack 86 | phr | 140 | 140 | 140 |
| Edelex 956 | | 40 | 85 | 40 |
| Irganox 1010 | | 3 | 3 | 3 |
| | | | | |
| calculated Tg | °C | -18 | -25 | -25 |
| | | | | |
| RBT at + 23°C | | 2 | 1.2 | 1 |
| RBT at + 15°C | | 3.5 | 1.7 | 2 |
| RBT at + 10°C | Cm | >40 | 2.3 | 3.5 |
| RBT at + 5°C | | | 6.3 | 19.5 |
| RBT at 0°C | | | >30 | >30 |
| | | | | |
| LT | N/25 mm | 17 | 12 | 15 |
| PA | N/25 mm | 15 | 8 | 14 |
| HP ss 1 kg | min | 660 | 216 | 1560 |
| HP ss 2 kg | min | 240 | 48 | 190 |
| SAFT | °C | 80 | 75 | 85 |

### Example 3

In label adhesives, SIS polymers are often blended with SB or SBS block copolymers to make the formulation softer and better suited for the label converting, namely the die-cutting and matrix stripping processes (US 5,663,228). However, it should be noticed that blends of polymers of the present invention, like Polymer A, B and C with SIS, SBS and SB are miscible and give only one tan delta peak as measured by Dynamic mechanical analysis.

Table 6 shows the Pressure Sensitive Adhesive results obtained for SIS/SB and Polymer B/SB and SBS

Results from Formulations F-7/F-8 show that an aliphatic resins is not good for a Polymer B/SB blend (F-8) as there is no tack measured neither by the Rolling ball Tack nor by the Loop Tack.

The combination of Polymer B/SB with MBG 223 (F-9) and Wingtack 86 (F-10) provide to the formulations good tack properties at lower temperature than SIS. It is also seen in Formulation F-11 compounded that with a paraffinic oil, having a lower glass transition temperature Tg than the naphtenic oil, that the Rolling ball tack values are excellent even at temperature as low as 0°C.

Properties obtained with blends of Polymer B/SB (F-11) and Polymer B/SBS (F-12) are similar with slightly better cohesion with the formulation F-12 because SBS is a triblock copolymer.

It should also be pointed out that the adhesive of Formulation F-11 has the characteristics of being a good removable adhesive. Applied on a paper surface, this adhesive can be easily removed even after a prolonged storage period.

**Table 6**

| Ingredients | Units | F-7 | F-8 | F-9 | F-10 | F-11 | F-12 comp |
|---|---|---|---|---|---|---|---|
| D-1113 | | 44 | | | | | |
| Polymer B | | | 44 | 44 | 44 | 44 | 44 |
| Solprene 1205 | | 56 | 56 | 56 | 56 | 56 | |
| Kraton D-1118 | phr | | | | | | 56 |
| Piccotac 1094 | | 100 | 100 | | | | |
| MBG 223 | | | | 100 | | | |
| Wingtack 86 | | | | | 100 | 100 | 100 |
| Edelex 956 | | 63 | 63 | 63 | 63 | | |
| Edelex 925 | | | | | | 63 | 63 |
| Irganox 1010 | | 3 | 3 | 3 | 3 | 3 | 3 |
| | | | | | | | |
| RBT at + 23°C | cm | 5 | >40 | 2 | 1.6 | 1.5 | 1.7 |
| RBT at +15°C | cm | 21 | | 3 | 2 | 2.2 | 2 |
| RBT at + 10°C | cm | >40 | | 8 | 1.7 | 2 | 2.3 |
| RBT at + 5 °C | cm | | | 8 | 2 | 2.1 | 2.3 |
| RBT at 0 °C | cm | | | n.m. | >30 | 7 | 9 |
| | | | | | | | |
| Loop tack | N/25 mm | 16 | 0 | 15 | 11 | 7 | 6 |
| Peel Adhesion | N/25 mm | 13 | 16 | 15 | 13 | 5.6 | 6 |
| HP 2 kg | hours | 0.3 | 0.4 | 0.5 | 12 | 6 | 18 |
| n.m. = not measured | | | | | | | |

## Claims

1. Adhesive composition for tapes, labels and bandages to be used at temperatures of +5°C and lower, comprising
a) at least one block copolymer, comprising at least two terminal blocks of poly(vinyl aromatic compound) and at least one midblock of a randomly copolymerized mixture of isoprene and butadiene,optionally mixed with a diblock copolymer comprising one poly(vinyl aromatic compound) block and one randomly copolymerized mixture of isoprene and butadiene, and optionally mixed with a block copolymer, comprising at least one block of poly(vinyl aromatic compound) and at least one block of poly(butadiene) or poly(isoprene),
b) at least one mixed aliphatic/aromatic hydrocarbon resin or a blend of aliphatic and aromatic hydrocarbon resins, having an aromatic H-NMR content between 6 and 22%, and
c) a plasticizer in an amount of at most 25 wt.%, relative to the weight of the adhesive composition.

2. Adhesive composition for tapes, labels and bandages according to claim 1, wherein component (a) mainly consists of a S-(I/B)-S or [S-(I/B]ₙX block copolymer, optionally mixed with a diblock copolymer S-(I/B), and optionally mixed with minor amounts of one or more block copolymers, selected from the group of S-B, S-B-S. S-I and S-I-S, wherein S represents a poly(vinyl aromatic compound) block, (I/B) represents a block of a randomly polymerized mixture of isoprene and butadiene, wherein the weight ratio between isoprene and butadiene is in the range of from 70:30 to 30:70, and wherein B represents a poly(butadiene) block, wherein I represents an poly(isoprene) block.

3. Adhesive composition according to claim 2 wherein the contents of S-B, S-I, S-B-S and/or S-I-S block copolymers is in the range of from 0 to 50 wt.%, relative to the weight of the component (a).

4. Adhesive composition according to claim 1 wherein component (b) has an aromatic H-NMR content between 9 and 22 % and more preferably from 9 to 18 %.

5. Adhesive composition according to claim 4 wherein component (b) is selected from the group consisting of or mixtures thereof.

6. Adhesive composition according to claims 1-5 wherein component (b) occurs in a proportion of from 35 to 55 wt.%, relative to the weight of the composition.

7. Adhesive composition according to claims 1-6 wherein the S blocks in the block copolymers of component (a) are poly(styrene) blocks and wherein the proportion of bound styrene in the main S-(I/B)-S or [S-(I/B)]ₙX block copolymers is in the range of from 10 to 40 wt.%.

8. Adhesive composition according to claims 1-7 wherein the (I/B) block in the main block copolymer has a vinyl content in the range of from 5 to 20 wt.%, based on the weight of the conjugated diene.

9. Tape, labels and bandages, comprising an adhesive composition according to claims 1-8, applied on a substrate layer.

10. Use of tapes, labels and bandages according to claim 9 at temperatures of +5°C or lower.
